# EUROPÄISCHE PATENTANMELDUNG

(11) **EP 4 427 795 A1**
(43) Veröffentlichungstag der Anmeldung: **11.09.2024**
(21) Anmeldenummer: 23161322.5
(22) Anmeldetag: 10.03.2023
(51) Int. Cl.: A61M 60/508, A61M 60/13, A61M 60/871

(54) **STEUEREINHEIT FÜR EINE BLUTPUMPE SOWIE BLUTPUMPENANORDNUNG**

(71) Anmelder: Berlin Heart GmbH, 12247 Berlin (DE)
(72) Erfinder: FRISCHKE, Dr.-Ing. Michael, 12247 Berlin (DE); BAUTZE, Thaddäus, 12247 Berlin (DE)
(74) Vertreter: Pfenning, Meinig & Partner mbB

(57) **Zusammenfassung**

Die Erfindung bezieht sich auf eine Steuereinheit (1) für eine Blutpumpe (2), umfassend:
ein Gehäuse (3),
einen ersten elektrischen Energiespeicher (4) und einen zweiten elektrischen Energiespeicher (5), die in dem Gehäuse angeordnet sind, und
eine mit einer Blutpumpe elektrisch koppelbare Übertragungsschnittstelle (6a, 6b) zur Versorgung der Blutpumpe mit elektrischer Energie; wobei die Steuereinheit dazu eingerichtet ist, elektrische Energie des ersten elektrischen Energiespeichers an der Übertragungsschnittstelle bereitzustellen. Durch den im Gehäuse angeordneten zweiten Energiespeicher wird eine signifikant erhöhte Energieversorgungssicherheit erreicht. Zudem bezieht sich die Erfindung auf eine Blutpumpenanordnung mit einer Blutpumpe und eine Steuereinheit der genannten Art.

## Beschreibung

Die Erfindung liegt auf dem Gebiet der Medizintechnik, insbesondere der Herzunterstützungssysteme und des elektrischen Apparatebaus.

In der Medizin sind Herzunterstützungssysteme mit Blutpumpen zur Aufrechterhaltung des Blutkreislaufs bekannt. Viele solche Pumpen sind zumindest teilweise implantierbar und auch transportabel, so dass Personen mit solchen Systemen zumindest teilweise mobil sind.

Blutpumpen haben im Betrieb einen durchaus beträchtlichen Energiebedarf und es sind Lösungen zur Energieversorgung, beispielsweise für VAD-(Ventricle Assist Devices) Pumpen bekannt, die sich teilweise Akkumulatoren, jedoch auch eines Netzanschlusses bedienen.

Soll eine derartige Pumpe möglichst problemlos auch mobil genutzt werden können, so ist die Sicherheit und Zuverlässigkeit der Energieversorgung unabdingbar. Es ist das Ziel eines Energieversorgungssystems für mobile Herzunterstützungssysteme, bestimmte Redundanzen sicherzustellen, um mit ausreichender Sicherheit die Unversehrtheit von Nutzern/Patienten zu gewährleisten. Dies wird derzeit bei einigen Beispielen des Standes der Technik durch eine Kombination von internen und extern anschließbaren elektrischen Energiequellen, insbesondere Energiespeichern, erreicht.

Beispielsweise ist aus der US- Patentschrift US 8394009B2 ein mobiles System bekannt, das einen im Gehäuse einer Steuereinheit verbauten aufladbaren Energiespeicher mit einem extern ansteckbaren Akkumulator verbindet.

Der vorliegenden Erfindung liegt vor dem Hintergrund des Standes der Technik die Aufgabe zugrunde, eine erhöhte Energieversorgungssicherheit von Herzunterstützungssystemen zu erreichen und dabei sowohl den Platzbedarf als auch das Gewicht von Energiespeichern möglichst gering zu halten.

Diese Aufgabe wird mit den Merkmalen der Erfindung gelöst durch eine Steuereinheit für eine Blutpumpe, umfassend:
ein Gehäuse, einen ersten elektrischen Energiespeicher und einen zweiten elektrischen Energiespeicher, die in dem Gehäuse angeordnet sind, und
eine mit einer Blutpumpe elektrisch koppelbare Übertragungsschnittstelle zur Versorgung der Blutpumpe mit elektrischer Energie; wobei die Steuereinheit dazu eingerichtet ist, elektrische Energie des ersten elektrischen Energiespeichers an der Übertragungsschnittstelle bereitzustellen.

Optional kann die Steuereinheit zusätzlich auch dazu eingerichtet sein, elektrische Energie des zweiten elektrischen Energiespeichers an der Übertragungsschnittstelle bereitzustellen. Es kann jedoch auch vorgesehen sein, dass die Steuereinheit dazu eingerichtet ist, innerhalb des Gehäuses ausschließlich den ersten Energiespeicher zur Bereitstellung von Energie an der Übertragungsschnittstelle zu verwenden. Der zweite Energiespeicher bleibt dann für andere Anwendungen reserviert.

Dadurch, dass innerhalb des Gehäuses zwei Energiespeicher vorgesehen und fest verbaut oder integriert sind, müssen und können diese im Normalbetrieb vom Patienten nicht gewechselt werden. Dadurch sind Bedienfehler beim Tausch, Einstecken oder der Ladung dieser Energiespeicher minimiert. Die Energiespeicher sind im Gehäuse gut geschützt und das Gehäuse kann beispielsweise fluiddicht, insbesondere wasserdicht ausgebildet sein. Beispielsweise kann das Gehäuse derart ausgestaltet sein, dass es eines Werkzeugs wie eines Schraubendrehers oder sogar eines an das Gehäuse angepassten Spezialwerkzeugs bedarf, um es zu öffnen.

Der Platzbedarf der Energiespeicher im Gehäuse ist gegenüber außen ansteckbaren Akkus deutlich reduziert und ebenso das Gewicht, da keine separate Umhüllung der Energiespeicher innerhalb des Gehäuses notwendig ist.

Bei einfach wechselbaren Energiespeichern tritt zudem oft eine Abnutzung von Steckkontakten oder sonstigen Verschleißteilen auf, so dass die Lebensdauer und/oder Zahl von Steckzyklen in solchen Fällen, ebenso wie die Vorschriftsmäßigkeit der gewechselten Energiespeicher, auch überwacht werden müsste. Bei den erfindungsgemäß genutzten Energiespeichern, die als Batterien, Kondensatoren oder aufladbare Akkus (Akkumulatoren) fest verbaut sind, sind solche Maßnahmen nicht notwendig. Auch die Notwendigkeit, im Zusammenhang mit den Energiespeichern und einem möglichen Tausch umfangreiche Selbsttests durchzuführen, entfällt. Durch das Vorhandensein von zwei oder mehr Energiespeichern in dem Gehäuse wird ein ausreichendes Maß an Energieversorgungssicherheit erreicht. Der erste und zweite Energiespeicher sind gemeinsam in einem einzigen Gehäuse untergebracht und befestigt, dessen Außenwände starr miteinander verbunden und gegeneinander unbeweglich sind. Das Gehäuse kann auch ganz oder teilweise implantierbar sein. Es kann einen Zugang zu den integrierten Energiespeichern aufweisen in Form einer Klappe oder eines Deckels, die im Normalbetrieb mit den übrigen Teilen des Gehäuses fest verbunden und in vielen Fällen nur unter Zuhilfenahme eines Werkzeugs zu öffnen sind. Die Energiespeicher können auch alternativ bündig und wasserdicht mit dem Gehäuse der Steuereinheit abschließen und an diesem festgeschraubt werden, so dass die Energiespeicher oder ihre Umhüllung einen Teil der Außenhaut der Steuereinheit bilden. Auch die integrierten Energiespeicher selbst können innerhalb des Gehäuses noch fixiert, beispielsweise eingeklemmt, eingeklebt oder verschraubt sein. Sie können jedoch grundsätzlich auch austauschbar sein. Der erste und/oder der zweite Energiespeicher können jeweils als Batterie, Akkumulator oder auch als Kondensator oder jede andere Form von Speicher für elektrische Energie ausgebildet sein. Beispielsweise ist eine Kombination denkbar, bei der der erste Energiespeicher als Akkumulator und der zweite Energiespeicher als aufladbarer elektrischer Kondensator ausgebildet ist. Es kann auch beispielsweise einer der Energiespeicher, insbesondere der zweite Energiespeicher oder es können beide Energiespeicher als Primärzellen/Batterien ausgebildet sein.

Die Übertragungsschnittstelle zur Übertragung von elektrischer Energie zur Blutpumpe kann ein elektrischer Leitungsanschluss sein, der auch steckbar/lösbar sein kann. Es kann jedoch auch eine Schnittstelle sein, bei der mittels elektrischer Felder, insbesondere Wechselfelder, Energie kontaktlos übertragen werden kann, beispielsweise durch eine magnetische Induktionsschleife.

Die Steuereinheit kann eine elektronische Einheit sein, die wenigstens einen Prozessor, insbesondere einen Microcontroller aufweisen kann. Die Steuereinheit steuert in vielen Fällen die Abgabe von Energie an die Blutpumpe sowie die Abgabe von Energie durch den ersten und zweiten Energiespeicher insgesamt, auch an Aggregate der Steuereinheit sowie, welcher von beiden Energiespeichern die Blutpumpe speist, wobei in manchen Ausführungsformen auch beide Energiespeicher gleichzeitig die Blutpumpe versorgen können. Die Steuereinheit kann zudem die Zufuhr von elektrischer Energie an die Steuereinheit sowie von externen Energiequellen an jeden der beiden oder auch weiterer integrierter Energiespeicher für deren Aufladung steuern.

Bei einer Implementierung der Steuereinheit kann beispielsweise vorgesehen sein, dass der erste elektrische Energiespeicher dieselbe Speicherkapazität hat wie der zweite elektrische Energiespeicher oder dass der zweite elektrische Energiespeicher eine geringere Speicherkapazität hat als der erste elektrische Energiespeicher, insbesondere weniger als 70%, weiter insbesondere weniger als 50%, weiter insbesondere weniger als 10% der Speicherkapazität des ersten elektrischen Energiespeichers.

Der zweite Energiespeicher kann auch beispielsweise weniger als 1 % der Kapazität des ersten Energiespeichers aufweisen.

In vielen Fällen ist es nützlich, wenn der erste Energiespeicher grundsätzlich für die Versorgung der Blutpumpe vorgesehen ist und diese auch wenigstens einige Stunden, beispielsweise wenigstens 2 oder wenigstens 5 Stunden versorgen kann.

In vielen Fällen ist es zudem nützlich, wenn zudem der zweite Energiespeicher für kurze Zeit, beispielsweise wenigstens 2 Minuten, wenigstens 10 oder 15 oder 30 Minuten, insbesondere aber für weniger als 1 Stunde, oder auch für weniger als 10 Minuten die Energieversorgung für die Blutpumpe leisten kann.

In einigen Fällen kann es auch sinnvoll sein, dass der zweite Energiespeicher ausschließlich zur Versorgung anderer Aggregate mit Ausnahme der Blutpumpe vorgesehen und die Steuereinheit dementsprechend eingerichtet ist.

Beispielsweise kann die zweite Energiespeichereinheit ausschließlich mit einer Alarmeinheit und/oder ausschließlich mit einer Notalarmeinheit verbunden oder verbindbar sein. Der erste Energiespeicher kann dagegen mit allen Aggregaten der Steuereinheit und mit der Blutpumpe verbunden oder verbindbar sein. Das Vorhandensein des zweiten Energiespeichers würde dann im Unterschied zu einem Zustand mit nur einem einzigen Energiespeicher einen großen Nutzen dadurch entwickeln, dass bei Ausfall des ersten Energiespeichers der Nutzer alarmiert würde oder ein Notalarm ausgelöst werden und ein Notalarmsignal ausgegeben werden könnte.

Bei einer Steuereinheit mit einer Notalarmeinheit, die dazu eingerichtet ist, ein Notalarmsignal zu erzeugen, wenn eine Notfallbedingung erfüllt ist, kann aber zudem auch vorgesehen sein, dass
die Steuereinheit dazu eingerichtet ist, die Notalarmeinheit mit Energie des ersten elektrischen Energiespeichers und insbesondere auch mit Energie des zweiten elektrischen Energiespeichers zu versorgen.

Die Steuereinheit kann dann die Notalarmeinheit in Abhängigkeit vom der Kapazität der Energiespeicher mit dem ersten und/oder dem zweiten Energiespeicher verbinden. Fällt einer der Energiespeicher aus, so kann somit der andere Energiespeicher noch die Notalarmeinheit versorgen.

Die Notalarmeinheit kann zusätzlich zu einer Alarmeinheit in der Steuereinheit vorgesehen sein und im Unterschied zu der Alarmeinheit andere/zusätzliche Funktionen erfüllen. Beispielsweise kann die Alarmeinheit allgemein den Nutzer oder Dritte auf einen Zustand der Steuereinheit oder der Blutpumpe hinweisen, der früher oder später ein Eingreifen erfordert oder bestimmte Handlungsoptionen ermöglicht. Dazu kann die Alarmeinheit verschiedenartige akustische und/oder optische Signale ausgeben. Die Notalarmeinheit ist grundsätzlich für die Ausgabe von dringlicheren Signalen vorgesehen, die ein zeitlich dringliches Eingreifen erfordern oder auf einen Funktionsverlust eines Aggregats, insbesondere auch der Alarmeinheit, hinweisen und/der die ein erhöhtes Risiko für die Sicherheit des Nutzers anzeigen. Die Notalarmeinheit kann einen deutlich geringeren Funktionsumfang und eine geringere Zahl und Differenziertheit möglicher ausgebbarer Signale aufweisen als die Alarmeinheit und somit auch einen deutlich geringeren Energieverbrauch während ihrer Funktion.

Bei einer Steuereinheit der beschriebenen Art mit einer Notalarmeinheit, die dazu eingerichtet ist, ein Notalarmsignal zu erzeugen, wenn eine Notfallbedingung erfüllt ist, kann zudem vorgesehen sein, dass die Steuereinheit eine Energiesteuereinheit sowie eine Funktionssteuereinheit aufweist, dass die Energiesteuereinheit dazu eingerichtet ist, die Notalarmeinheit aus dem ersten und/oder dem zweiten elektrischen Energiespeicher zu versorgen und dass der zweite elektrische Energiespeicher unmittelbar mit der Notalarmeinheit verbunden und dazu eingerichtet ist, die Notalarmeinheit auch unabhängig von der Steuerung durch die Energiesteuereinheit mit Energie zu versorgen.

Durch diese Ausgestaltung der Steuereinheit kann bei einem Ausfall der Energiesteuereinheit und/oder der Funktionssteuereinheit der zweite Energiespeicher die Notalarmeinheit immer noch unabhängig von den genannten Elementen mit Energie versorgen, so dass die Ausgabe eines Notalarms immer noch möglich ist.

Innerhalb der Steuereinheit können die Energiesteuereinheit und die Funktionssteuereinheit als separate Schaltungskomponenten eines elektronischen Gerätes ausgeführt sein, jedoch auch als Funktionseinheiten einer digitalen Steuerung, insbesondere als separate Funktionseinheiten eines Datenverarbeitungsprogramms, die jeweils separat bestimmte Gruppen von elektronischen Messsignalen verarbeiten und bestimmte Ausgangssignale erzeugen können.

Die Steuereinheit kann auch eine Unterbrechereinheit aufweisen, die dazu eingerichtet ist, einen aktiven Zustand oder einen inaktiven Zustand anzunehmen, und im aktiven Zustand ein Deaktivierungssignal bereitzustellen, welches bewirkt, dass das Erzeugen des Notalarmsignals verhindert wird.

Mit der genannten Unterbrechereinheit kann sichergestellt werden, dass der Notalarm auch bewusst abgestellt werden kann, beispielsweise vor dem Ausschalten der Steuereinheit und /oder der Blutpumpe. Die Unterbrechereinheit kann auch während der Herstellung der Steuereinheit oder ihrer Bestückung mit Energiespeichern oder während einer Lagerung der Steuereinheit aktiviert sein. Das Deaktivierungssignal kann auch bereitgestellt und beispielsweise außer an die Notalarmeinheit auch an die Funktionssteuereinheit übermittelt werden, um beim Ausschalten der Steuereinheit das automatische Zuschalten von Energiequellen, z.B. durch eine analoge Diodenschaltung, zu unterdrücken.

Die Steuereinheit kann auch eine Selbsttesteinrichtung aufweisen, wobei die Notalarmeinheit dazu eingerichtet ist, beim Empfang eines Selbsttestsignals unabhängig von der Notfallbedingung das Notalarmsignal zu erzeugen.

Die Selbsttesteinrichtung kann manuell oder automatisch ausgelöst werden, um das Notalarmsignal selbst zu testen.

Eine mögliche Implementierung der Erfindung kann vorsehen, dass die Steuereinheit dazu eingerichtet ist, die Erfüllung einer Notfallbedingung dann zu signalisieren, wenn der erste und/oder der zweite elektrische Energiespeicher einen Zustand erreicht, in dem seine Leistungsfähigkeit nicht ausreicht, zumindest jede der im zugeordneten Funktionen einzeln oder bestimmte Gruppen von Funktionen gleichzeitig zu erfüllen. Alternativ zu einer Funktionskontrolle kann für die Prüfung von Notfallbedingungen auch die Überwachung der zur Verfügung gestellten Spannung von Energiequellen, insbesondere Energiespeichern, verwendet werden und die Prüfung, ob diese vorschriftsgemäß oberhalb von bestimmten Schwellwerten liegen.

Wichtig ist beispielsweise in einigen Ausführungsformen, dass die Notfallbedingung erfüllt ist und ein entsprechendes Notfallsignal abgegeben wird, sobald der erste Energiespeicher nicht mehr ausreichend Kapazität hat, um den Betrieb der Blutpumpe zu gewährleisten. Sind sowohl der erste als auch der zweite Energiespeicher zur Versorgung der Blutpumpe vorgesehen, so kann die Notfallbedingung erfüllt sein, wenn wenigstens einer der Energiespeicher nicht mehr ausreichend Kapazität hat, um den Betrieb der Blutpumpe zu gewährleisten. In diesem Fall besteht die akute Gefahr, dass bei Ausfall von nur einem weiteren Energiespeicher die Funktion der Blutpumpe komplett ausfällt.

Sind den einzelnen Energiespeichern nur bestimmte Funktionen zur Energieversorgung von bestimmten Aggregaten der Steuereinheit und der Blutpumpe zugeordnet, so können den einzelnen Funktionen oder Aggregaten der Steuereinheit verschiedene Sicherheitsstufen der Energieversorgung zugeordnet werden, indem jeder Funktion der ersten oder der zweite Energiespeicher oder beide oder noch weitere Energiespeicher zur Versorgung zugeordnet werden. Ist das für eine bestimmte Funktion individuell geforderte Maß an Redundanz der Energieversorgung nicht gewährleistet, so ist eine Notfallbedingung erfüllt und es wird ein Notalarmsignal ausgegeben.

Es kann zudem vorgesehen sein, dass die Steuereinheit dazu eingerichtet ist, eine Erfüllung der Notfallbedingung regelmäßig, insbesondere wenigstens einmal pro Minute oder mittels einer elektronischen Analogschaltung ständig zu überprüfen.

Bei einer Steuereinheit der beschriebenen Art kann auch vorgesehen sein, dass die Steuereinrichtung eine Funktionssteuereinheit, insbesondere mit einem Hauptprozessor, umfasst und die Notfallbedingung erfüllt ist, wenn eine Fehlfunktion der Funktionssteuereinheit detektiert wird.

In diesem Fall kann oft die Funktionssteuereinheit den Notalarm nicht mehr ansteuern und auch die Energieversorgung der Notalarmeinheit über die Energiesteuerung nicht mehr veranlassen. Die Funktionssteuereinheit wird deshalb überwacht, beispielsweises mittels eines Watchdog, der die Erfüllung bestimmter Standardaufgaben durch die Funktionssteuereinheit bezüglich der Ergebnisse und/oder der benötigten Zeit überwacht. Die Watchdogfunktion kann selbständig die Notalarmeinheit ansteuern.

Für eine Steuereinheit mit einer Alarmeinheit, die ausgebildet ist, wenn eine Alarmbedingung erfüllt ist, ein Alarmsignal zu erzeugen, kann auch vorgesehen sein, dass insbesondere dann eine Notfallbedingung erfüllt ist, wenn eine Alarmbedingung für eine vordefinierte Zeitspanne ununterbrochen erfüllt ist.

Somit kann auch die Alarmfunktion überwacht und ihre Fehlfunktion durch die Notalarmeinheit signalisiert werden.

Für eine Steuereinheit der beschriebenen Art mit einer Energiesteuereinheit und einer Funktionssteuereinheit, kann auch vorgesehen sein, dass die Energiesteuereinheit mit der Übertragungsschnittstelle elektrisch verbunden oder mittels der Funktionssteuereinheit verbindbar und dazu eingerichtet ist, an der Übertragungsschnittstelle elektrische Energie des ersten elektrischen Energiespeichers und/oder des zweiten elektrischen Energiespeichers bereitzustellen.

Für eine Steuereinheit der beschriebenen Art mit einem oder mehreren Anschlüssen zur Verbindung jeweils einer externen elektrischen Energiequelle mit der Steuereinheit kann auch vorgesehen sein, dass die Steuereinheit dazu eingerichtet ist, eine durch eine externe Energiequelle bereitgestellte elektrische Energie zur Versorgung der Blutpumpe an der Übertragungsschnittstelle bereitzustellen.

Die Energiesteuereinheit ist in vielen Fällen zumindest mit dem ersten und zweiten Energiespeicher innerhalb des Gehäuses und potenziell auch noch mit weiteren Energiespeichern und/oder Energiequellen verbunden, so dass die Energiesteuereinheit, entweder gesteuert durch die Funktionssteuereinheit oder auch autonom in der Lage ist, Energie selektiv aus der ersten und/oder dem zweiten Energiespeicher oder von einer anderen, insbesondere externen Energiequelle, wie beispielsweise einem mit der Steuereinheit lösbar verbindbaren Akkumulator oder einem Netzanschluss/Netzteil an die Übertragungsschnittstelle zu übertragen und dort für die Blutpumpe bereitzustellen.

Für die Entscheidung der Energiesteuereinheit, von welchen Energiequellen und Energiespeichern zu einer bestimmten Zeit elektrische Energie an die Übertragungsschnittstelle geleitet wird, können bestimmte Vorgaben gemacht werden. Beispielsweise kann zunächst und mit Priorität, solange diese möglich ist, vor Inanspruchnahme der in das Gehäuse integrierten Energiespeicher Energie ausschließlich von Energiequellen außerhalb des Gehäuses zu der Übertragungsschnittstelle geleitet und für interne Funktionen der Steuereinheit verwendet werden. Es kann erst dann, wenn Energie aus externen Energiequellen nicht zur Verfügung steht, Energie aus dem ersten und zweiten Energiespeicher entnommen und zur Übertragungsschnittstelle geleitet oder für interne Funktionen der Steuereinheit verwendet werden.

Für eine Steuereinheit kann weiter vorgesehen sein, dass eine Energiesteuereinheit mit dem wenigstens einen Anschluss für eine externe Energiequelle elektrisch verbunden und dazu eingerichtet ist, wenn eine externe elektrische Energiequelle mit dem Anschluss verbunden ist, an der Übertragungsschnittstelle bevorzugt elektrische Energie der externen elektrischen Energiequelle bereitzustellen.

Solange Energie von externen Energiequellen durch die Steuereinheit bezogen wird, kann diese auch zum Aufladen an den ersten und/oder zweiten Energiespeicher geliefert werden.

Sind mehrere externe Energiequellen an die Steuereinheit angeschlossen, so kann, solange möglich, Energie von einem Netzanschluss bezogen werden, bevor ein Energiebezug von einem externen Akkumulator begonnen wird.

Zudem kann auch das Aufladen der im Gehäuse angeordneten Energiespeicher durch die externen Energiequellen durch die Energiesteuereinheit gesteuert werden.

Schließlich kann für eine Steuereinheit auch vorgesehen sein, dass das Gehäuse der Steuereinheit einen Zugang zum Gehäuseinnenraum für das Einsetzen und/oder einen Wechsel wenigstens eines der beiden elektrischen Energiespeicher umfasst.

Durch einen solchen Zugang ist zumindest durch Fachleute und/oder werkseitig der Tausch des ersten und/oder zweiten Energiespeichers möglich. Es kann auch vorgesehen sein, dass der erste und der zweite Energiespeicher und in manchen Fällen auch noch weitere Energiespeicher in einem gemeinsamen Untergehäuse angeordnet sind, das innerhalb des Gehäuses der Steuereinheit untergebracht und gegebenenfalls befestigt sein kann. Dieses Untergehäuse kann auch für diese Verwendung Steckkontakte aufweisen. Das Untergehäuse mit den Energiespeichern ist dann leicht tauschbar. Es kann auch jeder der Energiespeicher ein eigenes Untergehäuse aufweisen. Die Energiespeicher sind durch diese zusätzlichen Untergehäuse auch bei einem Tausch geschützt.

Weiter kann die Erfindung durch eine Blutpumpenanordnung realisiert werden, die eine Blutpumpe und eine Steuereinheit der oben beschriebenen Art umfasst, wobei die Blutpumpe mit der Steuereinheit über die Übertragungsschnittstelle elektrisch verbunden ist.

Die Steuerung des Energiebezugs von verschiedenen Energiequellen für den Betrieb der Blutpumpe oder den Betrieb von internen Aggregaten der Steuereinheit kann entweder durch analoge Elektronikschaltungen realisiert sein, insbesondere durch Diodenschaltungen oder eine ideale Diodenschaltung, die höheren zur Verfügung stehenden Versorgungsspannungen von einzelnen Energiequellen vor geringeren Spannungen anderer Energiequellen den Vorzug einräumen, oder durch Logikbausteine, insbesondere auch digital gesteuerte Elemente, die bestimmte Spannungen an den einzelnen Energiequellen messen, diese mit Spannungsschwellwerten für die einzelnen Energiequellen unter Nutzung einer digitalen Datenverarbeitung vergleichen und in Abhängigkeit von gemessenen Spannungen und dem Vergleich mit den Schwellwerten einzelne elektrische Leitungen sperren oder öffnen und damit einzelne Energiespeicher oder Energiequellen oder auch Gruppen von Energiespeichern gleichzeitig mit der Übertragungsschnittstelle und/oder einzelnen zu speisenden Aggregaten der Steuereinheit verbinden.

### Besondere Aspekte der Erfindung

Ein erster zusätzlicher Aspekt der Erfindung betrifft eine Blutpumpeneinrichtung, mit:
einer implantierbaren Blutpumpe, und
einer implantierbaren Steuereinheit, die über ein Kabel elektrisch mit der Blutpumpe verbunden ist, wobei
die implantierbare Steuereinheit zur Versorgung der Blutpumpe mit elektrischer Energie einen ersten elektrischen Energiespeicher, einen zweiten elektrischen Energiespeicher und eine Empfangseinheit zum kontaktlosen, insbesondere induktiven, Empfang von elektrischer Energie aufweist.

Bei dieser Konstellation ist durch den ersten und den zweiten Energiespeicher ein hohes Maß an Funktionssicherheit garantiert, auch wenn über die Energieempfangseinheit keine Energie von externen Energiequellen empfangen wird, da beispielsweise die Spule defekt und/oder kein Energiesender verfügbar ist, und einer der beiden Energiespeicher ausfällt.

Ein zweiter Aspekt der Erfindung betrifft eine Blutpumpeneinrichtung gemäß Aspekt 1, mit einer extrakorporale Energieversorgungseinheit, wobei
die extrakorporale Energieversorgungseinheit eine Sendeeinheit und eine elektrische Energiequelle aufweist, wobei die extrakorporale Energieversorgungseinheit dazu eingerichtet ist, elektrische Energie von der Energiequelle mittels der Sendeeinheit kontaktlos an die implantierbare Steuereinheit zu übertragen, und wobei die Energiequelle der extrakorporalen Energieversorgungseinheit insbesondere einen elektrischen Energiespeicher aufweist.

Ein dritter Aspekt der Erfindung betrifft eine Blutpumpeneinrichtung gemäß Aspekt 1 oder 2, wobei die extrakorporale Energieversorgungseinheit einen elektrischen Anschluss umfasst, um eine externe Energiequelle mit der extrakorporalen Energieversorgungseinheit elektrisch zu verbinden und die extrakorporale Energieversorgungseinheit dazu eingerichtet ist, eine durch die externe Energiequelle bereitgestellte elektrische Energie für die Versorgung der Steuereinheit und insbesondere der Blutpumpe, mit elektrischer Energie zu verwenden.

Ein vierter Aspekt der Erfindung betrifft eine Blutpumpeneinrichtung gemäß einem der vorhergehenden Aspekte, umfassend eine Notalarmeinheit, die ausgebildet ist, mittels einer Signaleinheit ein Notalarmsignal zu erzeugen, wenn eine Notfallbedingung erfüllt ist, wobei
die Blutpumpeneinrichtung ausgebildet ist, die Notalarmeinheit mit elektrischer Energie des ersten und/oder zweiten elektrischen Energiespeichers zu versorgen.

Im Folgenden wird die Erfindung anhand von Ausführungsbeispielen in Figuren einer Zeichnung gezeigt und nachfolgend erläutert.

Dabei zeigt
- Figur 1:: schematisch eine Steuereinheit gemäß der Erfindung mit den Merkmalen der Energieversorgung und der elektrischen Leitungen, die Energie zur Versorgung der einzelnen Aggregate führen,
- Figur 2:: schematisch eine Steuereinheit, wobei der Schwerpunkt der Darstellung auf dem Austausch von Signalen und Informationen zwischen den einzelnen Aggregaten liegt,
- Figur 3:: eine konkrete Darstellung eines beispielhaften Energieversorgungsschemas,
- Figur 4:: eine beispielhafte Darstellung von Alarmfunktionen einer Steuereinheit,
- Figur 5:: eine weitere Darstellung eines Energieversorgungsschemas einer Steuereinheit, sowie
- Figur 6:: eine Darstellung einer Steuereinheit mit einer transkutanen Übertragungsschnittstelle.

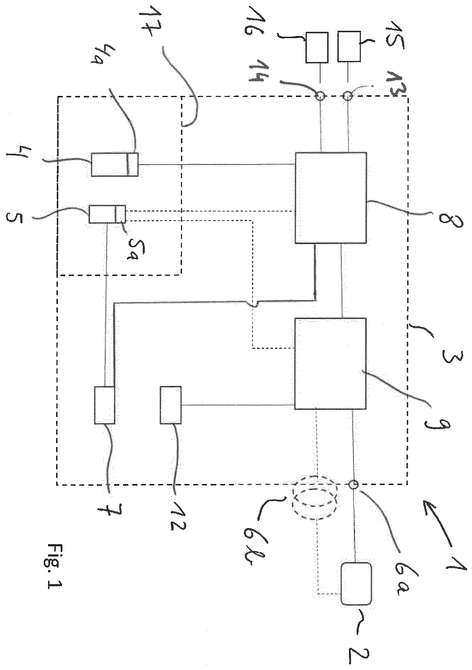

Die Figur 1 zeigt schematisch einen möglichen Aufbau einer Steuereinheit 1 für eine Blutpumpe 2, die beispielsweise als VAD- (Ventricular Assist Device) Pumpe, jedoch auch allgemein als Blutpumpe, vorzugsweise in implantierbarer Form, ausgeführt sein kann. Dabei erhebt die Figur keinen Anspruch auf Vollständigkeit und es soll anhand der Darstellung im Wesentlichen die Energieversorgung der einzelnen Aggregate der Steuereinheit und die Energieversorgung der Blutpumpe 2 erläutert werden. Innerhalb des Gehäuses 3, das fluiddicht und/oder auch implantierbar ausgeführt sein kann, sind zwei aufladbare elektrische Energiespeicher, und zwar ein erster Energiespeicher 4 und ein zweiter Energiespeicher 5, angeordnet und fest verbaut/integriert. Die Energiespeicher sind unter Verwendung eines Werkzeugs, insbesondere ausschließlich unter Verwendung eines Werkzeugs, durch Öffnen eines Gehäusedeckelteils 17 zugänglich, so dass werkseitig oder durch einen Fachmann ein Tausch der Energiespeicher vorgenommen werden kann. In der Steuereinheit 1 ist eine Energiesteuereinheit 8 vorgesehen, die beispielsweise mittels einer analogen Schaltung unter Verwendung von Dioden oder idealen Diodenschaltungen oder auch durch eine digitale Logikschaltungsanordnung die elektrischen Energieflüsse innerhalb der Steuereinheit, in diese hinein und von dieser aus zu der Blutpumpe 2 verwaltet und, teilweise gemeinsam mit der Funktionssteuereinheit 9 steuert. Die Funktionssteuereinheit kann externe Signale oder Befehlseingaben, sowie Messsignale von Sensoren innerhalb und außerhalb der Steuereinheit verarbeiten, Signale von der Blutpumpe empfangen und verarbeiten und diese Signale verknüpfen sowie Steuersignale für die Ansteuerung der Blutpumpe oder einzelner Aggregate der Steuereinheit erzeugen und ausgeben.

An die Steuereinheit 1 sind mittels Steckverbindungen externe Energiequellen 15, 16 ankoppelbar, wobei 15 beispielhaft einen Netzanschluss symbolisiert, während 16 einen ansteckbaren elektrischen Akkumulator darstellt. Innerhalb der Energiesteuereinheit kann durch eine analoge Schaltung allein aufgrund der an den einzelnen Verbindungsleitungen anliegenden Versorgungsspannungen, beispielsweise durch eine Kombination von Dioden, jeweils die passende Energieversorgung für alle oder einzelne Aggregate der Steuereinheit und für die Blutpumpe selektiert werden. Dies kann jedoch auch durch eine Logikschaltung erfolgen, die unter Verwendung digitaler Verarbeitung von Signalen die einzelnen Spannungsniveaus ermittelt und unter Berücksichtigung von bestimmten Prioritäten die Versorgung durch bestimmte Energiequellen unter den zur Verfügung stehenden Energiequellen abruft. Dabei kann vorgesehen sein, dass vorrangig, wenn vorhanden, Energie vom externen Netzanschluss bezogen wird, wenn dieser nicht angeschlossen ist von dem externen Akkumulator, mit nachfolgender Priorität von dem größeren der beiden internen Energiespeicher 4, 5 oder generell vom ersten Energiespeicher 4 und zuletzt von dem kleineren der beiden Energiespeicher 4, 5 oder von dem zweiten Energiespeicher 5. Es kann auch in bestimmten Kombinationen von zwei verschiedenen der Energiequellen, beispielsweise vom ersten und zweiten Energiespeicher 4, 5 parallel zueinander und gleichzeitig Energie bezogen werden. Die Entscheidung der Energiesteuereinheit, von welcher Energiequelle Energie bezogen wird, wird beispielsweise unmittelbar aus den zur Verfügung gestellten elektrischen Spannungen ermittelt oder aus übermittelten Sekundärinformationen wie beispielsweise einem separat durch Sensoren oder eine Entladungshistorie ermittelten Füllstand von Energiespeichern oder durch eine Kombination aller genannten Informationen. Hierzu können beispielsweise auch unmittelbar an den Energiespeichern 4, 5 Sensoren 4a, 5a zur Ermittlung von Füllständen oder für eine Billanzierung der ein- und ausfließenden elektrischen Ströme vorgesehen sein.

Außer dem Bezug von Energie aus den einzelnen Energiequellen steuert die Energiesteuereinheit auch die Weiterleitung der elektrischen Energie an die einzelnen Aggregate der Steuereinheit sowie an die Blutpumpe- in einigen Fällen in Zusammenarbeit mit der Funktionssteuereinheit.

Die Energiesteuereinheit 8 kann mit der bezogenen elektrischen Energie/einem Strom und einer Spannung unmittelbar die Funktionssteuereinheit 9 und auch die Blutpumpe 2 versorgen. Weiter kann die Energiesteuereinheit auch die Alarmeinheit/Hauptalarmeinheit 12 und in vielen Fällen auch die Notalarmeinheit 7 mit Energie versorgen.

Die Energiesteuereinheit kann auch eine Aufladung des ersten und/oder zweiten Energiespeichers durch den Bezug von Energie aus dem externen Akkumulator 16 und/oder dem Netzanschluss/Netztrafo 15 steuern.

Dem zweiten Energiespeicher 5, der beispielsweise eine ebenso große Kapazität wie der erste Energiespeicher 4, jedoch auch eine kleinere Kapazität als dieser aufweisen kann, können bestimmte Aggregate der Steuereinheit auch zur unmittelbaren Versorgung mit Energie unabhängig von einer Funktion oder Entscheidung der Energiesteuereinheit und insbesondere auch unabhängig von einer Entscheidung der Funktionssteuerung zugeordnet sein.

Dies kann beispielsweise die Notalarmeinheit 7 sein, die folglich auch unmittelbar von dem zweiten Energiespeicher elektrische Energie beziehen kann. Dem zweiten Energiespeicher können zusätzlich auch noch andere Aggregate der Steuereinheit derart zugeordnet sein, dass er diese auch unmittelbar ohne Mittwirkung der Energiesteuereinheit mit elektrischer Energie versorgen kann, beispielsweise eine Hauptalarmeinheit. In manchen Fällen kann auch vorgesehen sein, dass der zweite Energiespeicher einzelne Aggregate, beispielsweise die Notalarmeinheit auch exklusiv unmittelbar mit Energie versorgt, in dem Sinn, dass diese, also beispielsweise die Notalarmeinheit ausschließlich unmittelbar von dem zweiten Energiespeicher elektrisch mit Energie versorgt sind.

Unter anderem kann auch die Funktionssteuerung 9 neben ihrer elektrischen Energieversorgung durch die Energiesteuereinheit unmittelbar durch den zweiten Energiespeicher 5 versorgbar sein, wie die gestrichelt dargestellte elektrische Leitungsverbindung in der Figur 1 darstellt. Damit kann eine erhöhte Versorgungssicherheit für die unmittelbar mit dem zweiten Energiespeicher 5 verbundenen Aggregate erreicht werden für den Fall, dass die Energiesteuereinheit nicht funktioniert und/oder der erste Energiespeicher leer oder defekt ist.

Die Figur 1 zeigt im Übrigen die Übertragungsschnittstelle 6a, 6b in zwei Varianten, als galvanisch verbundenen, kontaktvermittelten elektrischen Anschluss 6a und als induktive Energieübertragungsstrecke 6b (TET, Transcutanious Energy Transfer).

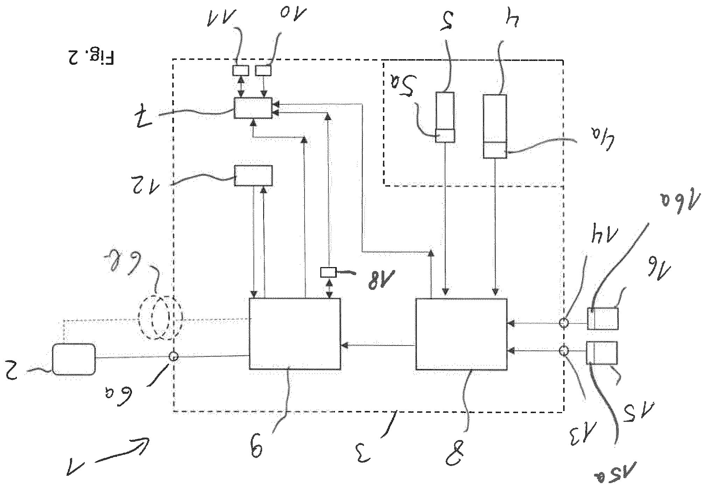

Die Figur 2 stellt vorwiegend die Verbindungen zwischen den Aggregaten der Steuereinheit intern und mit externen Elementen dar, die der Signalübertragung, der Befehlsübermittlung und/oder dem Informationsaustausch/der Informationsübertragung dienen. Beispielsweise können in einer Ausführungsform Informationen über den Füllstand oder die Verfügbarkeit von Energiequellen 4, 5, 15, 16 mittels einzelner Sensoren 4a, 5a, 15a, 16a jeweils an den einzelnen Energiequellen, also am ersten Energiespeicher 4, am zweiten Energiespeicher 5, an dem extern ansteckbaren Akkumulator 16 und am externen Netzanschluss 15 ermittelt und an die Energiesteuereinheit 8 übertragen werden. Die Energiesteuereinheit 8 kann jedoch auch selbst die anliegenden Spannungsniveaus der einzelnen elektrischen Energiequellen ermitteln und verarbeiten. Der Informationsaustausch findet in diesem letztgenannten Fall unmittelbar über die Energieleitungen statt. Anderenfalls können jedoch auch die in der Figur 2 gezeigten Verbindungen als parallele Signalleitungen verstanden werden und als elektrische oder optische Signalleitungen ausgeführt sein. Die in den Figuren 1 und 2 dargestellten Verbindungen sind rein funktional zu verstehen und können auch auf einzelnen Leitungen zusammengefasst sein, indem auf einer elektrischen Leitung sowohl Energie transportiert als auch Informationen in der Form von Signalen übermittelt werden können.

Die Energiesteuereinheit kann beispielsweise mit der Notalarmeinheit 7 über eine Signalleitung verbunden sein und dieser signalisieren, dass die Energieversorgung der Steuereinheit 9 und/oder der Blutpumpe 2 gefährdet ist, so dass die Notalarmeinheit ein Notalarmsignal ausgeben kann. Die Notalarmeinheit 7 ist auch mit der Funktionssteuerung 9 unmittelbar verbunden und kann von dieser die Anforderung eines Notalarmsignals, also einen Notalarmbefehl, empfangen. Die Funktionssteuereinheit kann zu diesem Zweck einzelne Aggregate der Steuereinheit überwachen. Notalarmsignale können somit auch unabhängig von der Funktionsfähigkeit der Energieversorgung erzeugt werden, beispielsweise auch, wenn die Blutpumpe selbst defekt ist. Diese kann auch durch die Funktionssteuereinheit überwacht werden und die Funktionssteuereinheit kann entweder Störsignale von der Blutpumpe oder auch einem anderen externen Aggregat empfangen oder einen Fehler über eine Änderung des Energiebedarfs detektieren. Zudem kann eine Selbstüberwachungseinheit 18, beispielsweise als Watchdogfunktion, vorgesehen sein, die die Funktionssteuerung überwacht und bei deren Funktionsausfall selbständig einen Notalarmbefehl an die Notalarmeinheit 7 ausgeben kann.

Die Funktionssteuereinheit 9 ist auch mit einer Alarmeinheit/Hauptalarmeinheit 12 verbunden, über die sie die Abgabe von bestimmten Hinweissignalen nach außen, beispielsweise für den Nutzer oder Dritte veranlassen kann. Die Alarmeinheit 12 ist dazu in der Lage, eine Mehrzahl von differenzierten akustischen und/oder optischen Signalen auszugeben, die auf verschiedene Situationen und/oder Zustände hinweisen können.

Das Vorliegen eines Alarms wird überwacht und es kann vorgesehen sein, dass das Vorliegen eines Alarms in ununterbrochener Folge für eine bestimmte Zeitdauer ein Notalarmsignal auslöst.

Die Notalarmeinheit 7 ist unter anderem auch mit einer Selbsttesteinrichtung 11 verbunden, die, entweder auf Anforderung von außen durch einen Nutzer, oder auch selbsttätig einen Notalarm der Notalarmeinheit auslösen und seine Funktion überprüfen kann.

Zudem ist die Notalarmeinheit mit einer Unterbrechereinheit 10 verbunden, die die Ausgabe eines Notalarmsignals unterbinden oder blockieren kann.

Diese Unterbrecherseinheit, die ein Deaktivierungssignal an die Notalarmeinheit senden kann, wird beispielsweise betätigt, wenn die Steuereinheit gebaut und mit Energiespeichern bestückt wird, zum Beispiel, wenn anfänglich die Steuereinheit mit nur einem Energiespeicher bestückt und danach gelagert wird. Zudem wird die Unterbrechereinheit auch vor dem Ausschalten der Steuereinheit betätigt.

Auch der zweite und/oder erste Energiespeicher 4, 5 kann unmittelbar über eine Signalleitung mit der Notalarmeinheit verbunden sein, um unabhängig von der Funktionsfähigkeit der anderen Aggregate der Steuereinheit ein Notalarmsignal auslösen zu können, falls der erste und/oder zweite Energiespeicher defekt oder vollständig entleert sein sollte.

Auch der Austausch von Informationen zwischen der Steuereinheit/der Energiesteuereinheit/der Funktionssteuereinheit einerseits und der Blutpumpe andererseits ist durch Signalleitungen ermöglicht, so dass beispielsweise auch ein Energie- oder Leistungsbedarf von der Blutpumpe signalisiert werden kann. Dieser Informationsaustausch kann über eine elektrische Leitung oder auch drahtlos über Funk oder über die Übertragungsschnittstelle erfolgen. Ein Leistungsbedarf der Blutpumpe kann allerdings auch unmittelbar durch die elektrische Leistungsabnahme, beispielsweise durch die Stromstärke des fließenden Stroms, der Funktionssteuereinheit signalisiert und von dieser analysiert werden.

Die Figur 2 zeigt, ebenso wie die Figur 1, die Übertragungsschnittstelle 6a, 6b in zwei Varianten, als galvanisch verbundenen, kontaktvermittelten elektrischen Anschluss 6a und als induktive Energieübertragungsstrecke 6b. An dieser Schnittstelle können parallel Energie und Information transportierende Signale übertragen werden.

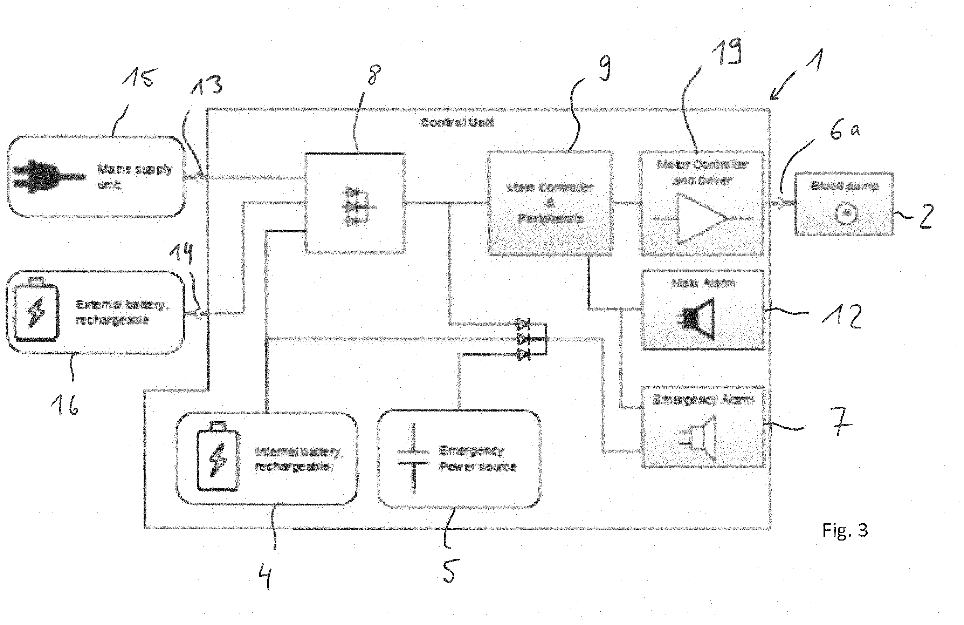

Figur 3 zeigt eine Steuereinheit 1 für eine VAD- Blutpumpe 2, an die mittels Steckverbindungen 13, 14 sowohl ein Netzteil 15 als Netzanschluss, als auch ein externer Akkumulator 16 jeweils einzeln oder gleichzeitig angeschlossen werden können. Zudem ist ein interner Akkumulator 4 als erster Energiespeicher leistungsfähig ausgebildet, so dass jede dieser genannten 3 Energiequellen für sich die Funktionssteuereinheit 9 mit einem Hauptprozessor, die Blutpumpe 2 und den Motortreiber 19 ausreichend mit elektrischer Energie versorgen kann. Der Motortreiber 19 kann beispielsweise eine Umrichtereinheit aufweisen, die durch die Funktionssteuerung mit Energie versorgt und angesteuert wird. Der Motortreiber kann dann unmittelbar Umrichtersignale, beispielsweise pulsweitenmodulierte Signale, an die Elemente des Motors der Blutpumpe ausgeben.

Der zweite Energiespeicher 5 ist als Notenergiequelle ausgebildet und betreibt in einem Beispielfall ausschließlich die Notalarmeinheit 7. Dadurch kann gewährleistet werden, dass der zweite Energiespeicher nicht durch andere Anwendungen vorzeitig entladen wird. Wenn alle anderen Energiequellen und/oder die Funktionssteuereinheit versagen, kann die Notalarmeinheit, versorgt durch den zweiten Energiespeicher, somit immer noch die Ausgabe eines Notalarmsignals bewirken.

Die Funktionssteuereinheit kann auch die Alarmeinheit/Hauptalarmeinheit 12 ansteuern, versorgt diese jedoch mit Energie, die über die Energiesteuereinheit von anderen Energiequellen bezogen wird, so dass die Hauptalarmeinheit, die aufgrund ihrer umfangreichen Funktionen einen deutlich höheren Energieverbrauch hat als die Notalarmeinheit 7, den zweiten Energiespeicher nicht in Anspruch nimmt.

In diesem Beispiel, wie auch in vielen anderen Fällen, kann der zweite Energiespeicher eine deutlich geringere elektrische Speicherkapazität aufweisen als der erste Energiespeicher und alternativ zu der Ausführung als aufladbarer Akkumulator auch beispielsweise als Knopfzelle oder als Speicherkondensator ausgeführt sein.

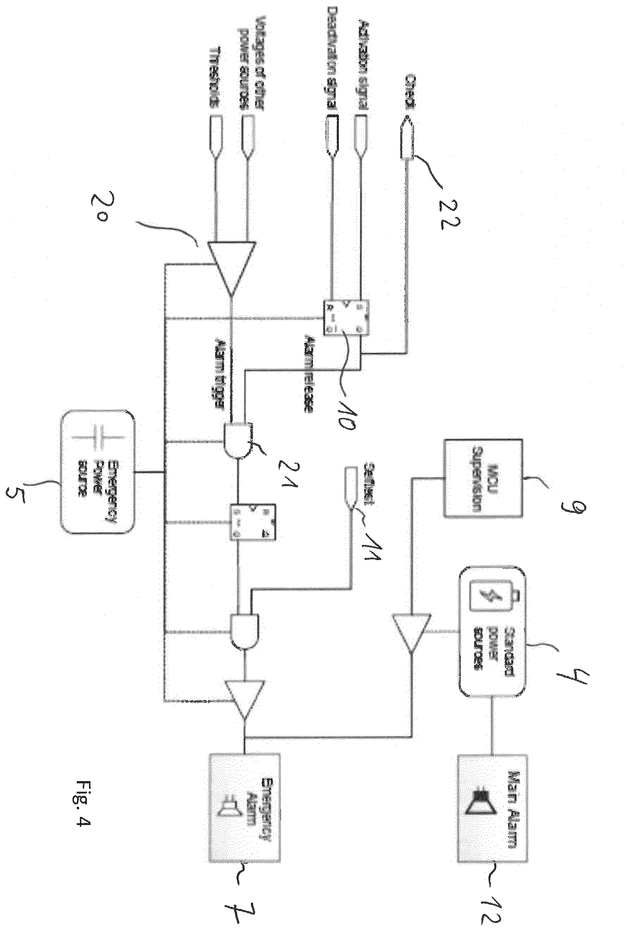

In der Figur 4 sind weitere Einzelheiten einer möglichen Gestaltung der Notalarmeinheit 7 und ihrer Peripherie dargestellt.

Der Vergleichseinheit 20 werden an ihren Eingängen die Spannungsniveaus der einzelnen angeschlossenen elektrischen Energiequellen 15, 16, 4, 5 zugeführt. Diese Niveaus werden mit einem Schwellwert oder mit individuell für jede Energiequelle festgelegten schwellwerten verglichen, der an einem weiteren Eingang anliegt und in dem Fall, dass alle Spannungsniveaus unter einem gemeinsamen kritischen Schwellwert oder jedes einzelne Spannungsniveau jeder angeschlossenen Energiequelle unter einem ihr individuell zugeordneten Schwellwert liegen, wird ein Notfallalarm ausgelöst und ein Notalarmbefehl an die Notalarmeinheit 7 geschickt. In der Prüfeinheit 21 wird zusätzlich überprüft, ob das Notalarmsignal durch die Unterbrechereinheit 10 deaktiviert ist. Ist dies der Fall, so wird die Weiterleitung des Notalarmbefehls an die Notalarmeinheit 7 blockiert.

Die Unterbrechereinheit 10 ist auch mit einer Selbsttesteinrichtung 22 versehen, die den Funktionstest der Unterbrechereinheit 10 ermöglicht. Wird eine Fehlfunktion der Unterbrechereinheit 10 festgestellt, so kann durch die Selbsttesteinrichtung 22 unmittelbar ein Notalarmbefehl an die Notalarmeinheit 7 geschickt werden.

Zudem ist eine Selbsttesteinrichtung 11 der Notalarmeinheit vorgesehen, die ohne einen Fehleranlaß erlaubt, einen Notalarmbefehl an die Notalarmeinheit 7 zu schicken. Es kann dann geprüft werden, ob ein Notalarmsignal ausgegeben wird oder ob die Notalarmeinheit 7 defekt ist.

Eine Energieversorgung der Notalarmeinheit kann nach diesem Beispiel durch den ersten Energiespeicher 4 ebenso wie durch den zweiten Energiespeicher 5 erfolgen. Der Hauptalarm 12 wird durch den ersten Energiespeicher 4 mit Energie versorgt, kann jedoch keine Energie von dem zweiten Energiespeicher 5 beziehen.

Der zweite Energiespeicher kann einen deutlich kleineren Bauraum einnehmen als der erste Energiespeicher, beispielsweise weniger als 70%, insbesondere weniger als 50%, weniger als 20% oder sogar weniger als 10% des Bauraums des ersten Energiespeichers. Der zweite Energiespeicher kann als Kondensator oder als elektronische Schaltung mit mehreren Kondensatoren ausgebildet sein, als kleiner aufladbarer Akkumulator oder als Primärzelle. Sein Gewicht kann beispielsweise weniger als 70%, insbesondere weniger als 50%, weniger als 20% oder sogar weniger als 10% des Gewichts des ersten Energiespeichers betragen.

Bei der Steuerung des Energiebezugs kann bevorzugt Energie von einem Netzanschluss und, wenn dieser nicht zur Verfügung steht, von einem ansteckbaren externen Akku bezogen werden. Sind beide nicht verfügbar, wird zunächst Energie aus dem ersten, größeren Energiespeicher 4 bezogen. Die Priorisierung der Quellen kann über die unterschiedlichen Spannungsniveaus der Energiequellen gesteuert werden. Dabei kann der Netzanschluss auf eine Betriebsspannung von 24VDC ausgelegt sein, der externe Akkumulator auf eine Spannung von 10 bis 17 V und der erste Energiespeicher 4 auf eine Spannung von 3 bis 5 Volt. Werden die Quellen mittels Dioden zusammengeschaltet, so ergibt sich automatisch eine Priorisierung des Energiebezugs in der oben genannten Reihenfolge.

Die Priorisierung kann mit verringertem Energiebedarf auch durch eine logische Schaltung erreicht werden, in der die Speisespannungen in digitalisierter Form erfasst und verarbeitet werden und aufgrund der Verarbeitung bestimmte Leitungen zu oder von einzelnen Energiequellen durchgeschaltet oder unterbrochen werden.

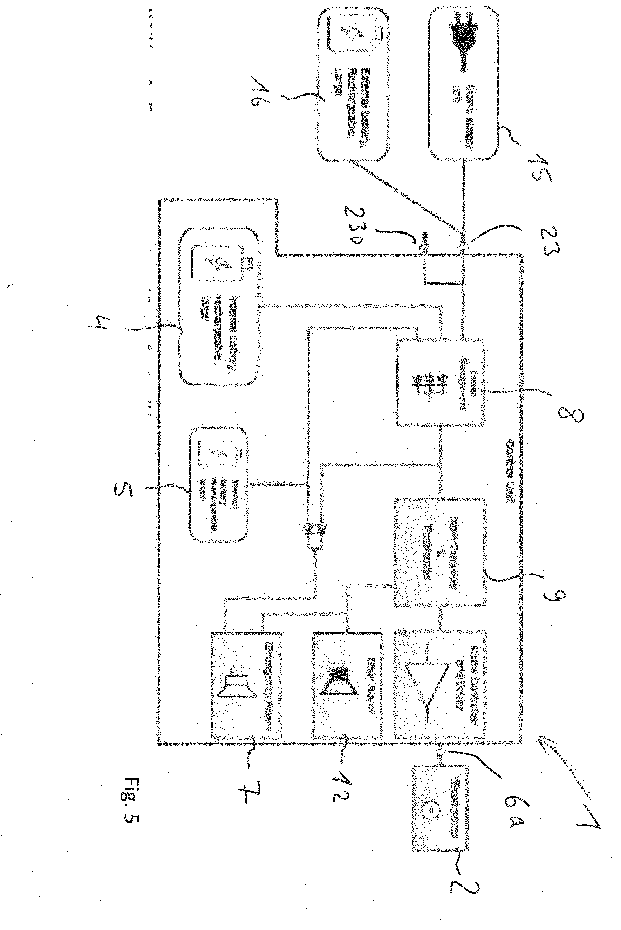

Die Figur 5 zeigt eine Steuereinheit 1, bei der der erste Energiespeicher Energie über die Energiesteuereinheit 8 einspeist, ebenso wie der zweite Energiespeicher 5. Diese Energie kann an verschiedene Aggregate der Steuereinheit 1 geleitet werden. Reicht eine Energieversorgung der Notalarmeinheit 7 durch die Energiesteuereinheit 8 nicht aus, so kann die Notalarmeinheit 7 auch unmittelbar durch den zweiten Energiespeicher 5 gespeist werden. Somit ist sichergestellt, dass die Versorgung der Notalarmeinheit funktioniert, solange einer der beiden Energiespeicher 4, 5 noch ausreichend Energie zur Verfügung hat und diese zur Verfügung stellt. Der Notalarm kann dann durch die jeweils noch funktionierenden Energiespeicher ausgelöst und der Notalarmbefehl an die Notalarmeinheit geschickt werden, Die beiden Energiespeicher 4, 5 können sich somit gegenseitig überwachen.

Bei der Ausführungsform der Figur 5 ist ein Anschluss 23 vorgesehen, an den wahlweise sowohl ein Akkumulator 16, als auch alternativ ein Netzteil 15 angeschlossen werden kann. Damit sind keine zwei verschiedenen Anschlüsse notwendig. Allerdings kann, um die Versorgungssicherheit zu erhöhen, der baugleiche Anschluss 23, 23a redundant nach außen zweimal zur Verfügung gestellt werden. Alternativ können auch zwei nicht baugleiche Anschlüsse zur Verfügung gestellt werden, die teilweise auch an bestimmte Energiequellen angepaßt sein können.

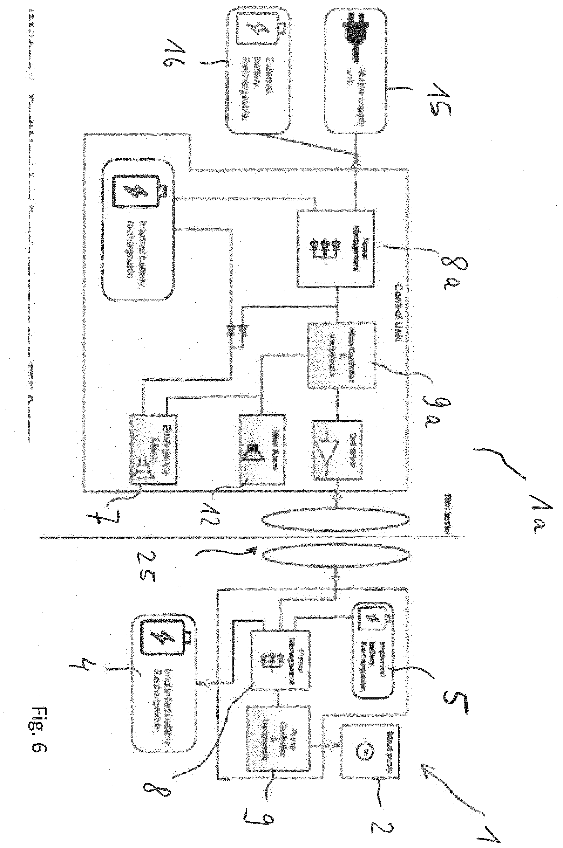

Die Figur 6 zeigt eine Implementierung der erfindungsgemäßen Steuereinheit 1 als implantierte Einheit mit einem ersten Energiespeicher 4 und einem zweiten Energiespeicher 5. Die Steuerung der Energieversorgung durch die Energiesteuereinheit 8 und die Funktionssteuereinheit 9 kann ebenso funktionieren, wie in den obigen Beispielen beschrieben. Allerdings kann die Steuereinheit 1 von einer externen, zweiten Steuereinheit 1a über eine kontaktlose Übertragungsschnittstelle in Form einer TET- Schnittstelle (Transcutaneous Energy Transfer) mittels induktiver Energieübertragung mit elektrischer Energie versorgt werden.

Wird die zweite Steuereinheit 1a entfernt, beispielsweise während der Benutzer duscht, so sind die beiden internen Energiespeicher 4, 5 der implantierten Steuereinhheit 1ausreichend, um einen Betrieb der Blutpumpe sowie eine Überwachung aller Funktionen einschließlich ihrer eigenen Funktion, wie bereits oben beschrieben zu gewährleisten.

Im Normalbetrieb wird die Blutpumpe mit der Energie betrieben, die von einem Netzgerät der zweiten Steuereinheit 1a geliefert und von dieser an die implantierte Steuereinheit mittels der TET- Kopplung übertragen wird. Ersatzweise kann auch Energie durch die zweite Steuereinheit von einem externen Akkumulator bezogen werden. Die zweite Steuereinheit kann mit einem ersten und einem zweiten Energiespeicher versehen sein und wie oben anhand der erläuterten Figuren bereits beschrieben funktionieren, jedoch kann, da ja bereits die implantierte Steuereinheit redundant und abgesichert mit Energie versorgt ist, innerhalb der zweiten Steuereinheit auch nur ein einziger Energiespeicher vorgesehen sein.

Es kann also bei einer derartigen Konstellation mit einem implantierbaren ersten Steuergerät für eine Blutpumpe, welches über eine TET- Übertragung mit einem extrakorporalen zweiten Steuergerät verbunden ist, vorgesehen sein, dass insgesamt nicht mehr als 3 interne Energiespeicher in beiden Steuergeräten vorgesehen sind, von denen eines der Steuergeräte zwei und das andere einen Energiespeicher enthält. Ein oder mehrere interne Akkumulatoren der extrakorporalen Steuereinheit können durch eine Spannungs-Vergleicherschaltung oder eine Logikschaltung auf ihren Ladezustand überwacht werden und im Falle des Unterschreitens einer Ladeschwelle durch einen oder mehrere der Akkumulatoren kann ein Notalarmbefehl an die Notalarmeinheit 7 ausgegeben werden, die dann ein Notalarmsignal ausgibt. Dies kann erfolgen, ohne dass die Funktionssteuereinheit 9a mit dem Hauptprozessor aktiviert wird.

Die implantierte Steuereinheit 1 kann ebenso aufgebaut sein wie die in den Figuren 1 bis 5 gezeigten Einheiten und kann insbesondere auch über eine separate Hauptalarmeinheit und eine Notalarmeinheit verfügen, die auch in der oben beschriebenen Art angesteuert werden können.

Verfügt die implantierte Steuereinheit 1 über eine Notalarmeinheit 7, so kann die externe zweite Steuereinheit in einigen Fällen auch ohne eine Notalarmeinheit aufgebaut werden und die Funktion, Notalarmsignale auszugeben, kann bei Überwachung beider Steuereinheiten durch die Notalarmeinheit der implantierten Steuereinheit übernommen werden.

## Patentansprüche

1. Steuereinheit (1) für eine Blutpumpe (2), umfassend:
ein Gehäuse (3),
einen ersten elektrischen Energiespeicher (4) und einen zweiten elektrischen Energiespeicher (5), die in dem Gehäuse angeordnet sind, und
eine mit einer Blutpumpe elektrisch koppelbare Übertragungsschnittstelle (6a, 6b) zur Versorgung der Blutpumpe mit elektrischer Energie; wobei die Steuereinheit dazu eingerichtet ist, elektrische Energie des ersten elektrischen Energiespeichers an der Übertragungsschnittstelle bereitzustellen.

2. Steuereinheit gemäß Anspruch 1, **dadurch gekennzeichnet, dass** der erste elektrische Energiespeicher (4) dieselbe Speicherkapazität hat wie der zweite elektrische Energiespeicher (5) oder dass der zweite elektrische Energiespeicher eine geringere Speicherkapazität hat als der erste elektrische Energiespeicher, insbesondere weniger als 70%, weiter insbesondere weniger als 50%, weiter insbesondere weniger als 10% der Speicherkapazität des ersten elektrischen Energiespeichers.

3. Steuereinheit gemäß Anspruch 1 oder 2, umfassend eine Notalarmeinheit (7), die dazu eingerichtet ist, ein Notalarmsignal zu erzeugen, wenn eine Notfallbedingung erfüllt ist, **dadurch gekennzeichnet, dass**
die Steuereinheit (1) dazu eingerichtet ist, die Notalarmeinheit mit Energie des ersten elektrischen Energiespeichers (4) und insbesondere auch mit Energie des zweiten elektrischen Energiespeichers (5) zu versorgen.

4. Steuereinheit gemäß einem der Ansprüche 1 bis 3, umfassend eine Notalarmeinheit (7), die dazu eingerichtet ist, ein Notalarmsignal zu erzeugen, wenn eine Notfallbedingung erfüllt ist, **dadurch gekennzeichnet, dass** die Steuereinheit (1) eine Energiesteuereinheit (8) sowie eine Funktionssteuereinheit (9) aufweist, dass die Energiesteuereinheit dazu eingerichtet ist, die Notalarmeinheit aus dem ersten und/oder dem zweiten elektrischen Energiespeicher (4, 5) zu versorgen und dass der zweite elektrische Energiespeicher unmittelbar mit der Notalarmeinheit verbunden und dazu eingerichtet ist, die Notalarmeinheit auch unabhängig von der Steuerung durch die Energiesteuereinheit mit Energie zu versorgen.

5. Steuereinheit gemäß einem der Ansprüche 1 bis 4, mit einer Unterbrechereinheit (10), die dazu eingerichtet ist, einen aktiven Zustand oder einen inaktiven Zustand anzunehmen, und im aktiven Zustand ein Deaktivierungssignal bereitzustellen, welches bewirkt, dass das Erzeugen des Notalarmsignals verhindert wird.

6. Steuereinheit gemäß einem der Ansprüche 3 bis 5, mit einer Selbsttesteinrichtung (11) wobei die Notalarmeinheit (7) dazu eingerichtet ist, beim Empfang eines Selbsttestsignals unabhängig von der Notfallbedingung das Notalarmsignal zu erzeugen.

7. Steuereinheit gemäß einem der Ansprüche 3 bis 6, **dadurch gekennzeichnet, dass** sie dazu eingerichtet ist, die Erfüllung einer Notfallbedingung dann zu signalisieren, wenn der erste und/oder der zweite elektrische Energiespeicher (4, 5) einen Zustand erreicht, in dem seine Leistungsfähigkeit nicht ausreicht, zumindest jede der im zugeordneten Funktionen einzeln oder bestimmte Gruppen von Funktionen gleichzeitig zu erfüllen.

8. Steuereinheit gemäß einem der Ansprüche 3 bis 7, **dadurch gekennzeichnet, dass** die Steuereinheit (1) dazu eingerichtet ist, eine Erfüllung der Notfallbedingung regelmäßig, insbesondere wenigstens einmal pro Minute oder mittels einer elektronischen Analogschaltung ständig zu überprüfen.

9. Steuereinheit gemäß einem der Ansprüche 3 bis 8, wobei die Steuereinheit eine Funktionssteuereinheit (9), insbesondere mit einem Hauptprozessor, umfasst und die Notfallbedingung erfüllt ist, wenn eine Fehlfunktion der Funktionssteuereinheit detektiert wird.

10. Steuereinheit gemäß einem der Ansprüche 1 bis 9, mit einer Hauptalarmeinheit (12), die ausgebildet ist, wenn eine Alarmbedingung erfüllt ist, ein Alarmsignal zu erzeugen, wobei insbesondere eine Notfallbedingung erfüllt ist, wenn eine Alarmbedingung für eine vordefinierte Zeitspanne ununterbrochen erfüllt ist.

11. Steuereinheit gemäß einem der vorhergehenden Ansprüche mit einer Energiesteuereinheit (8) und einer Funktionssteuereinheit (9), **dadurch gekennzeichnet, dass** die Energiesteuereinheit mit der Übertragungsschnittstelle (6a, 6b) elektrisch verbunden oder mittels der Funktionssteuereinheit verbindbar und dazu eingerichtet ist, an der Übertragungsschnittstelle elektrische Energie des ersten elektrischen Energiespeichers (4) und/oder des zweiten elektrischen Energiespeichers (5) bereitzustellen.

12. Steuereinheit (1) gemäß einem der vorhergehenden Ansprüche, mit einem oder mehreren Anschlüssen (13, 14) zur Verbindung jeweils einer externen elektrischen Energiequelle (15, 16) mit der Steuereinheit **dadurch gekennzeichnet, dass** die Steuereinheit dazu eingerichtet ist, eine durch eine externe Energiequelle bereitgestellte elektrische Energie zur Versorgung der Blutpumpe (2) an der Übertragungsschnittstelle (6a, 6b) bereitzustellen.

13. Steuereinrichtung gemäß Anspruch 12 **dadurch gekennzeichnet, dass** eine Energiesteuereinheit (8) mit dem wenigstens einen Anschluss (13, 14) für eine externe Energiequelle (15, 16) elektrisch verbunden und dazu eingerichtet ist, wenn eine externe elektrische Energiequelle mit dem Anschluss verbunden ist, an der Übertragungsschnittstelle (6a, 6b) bevorzugt elektrische Energie der externen elektrischen Energiequelle (15, 16) bereitzustellen.

14. Steuereinheit gemäß einem der Ansprüche 1 bis 13, **dadurch gekennzeichnet, dass** das Gehäuse (3) der Steuereinheit (1) einen Zugang zum Gehäuseinnenraum für das Einsetzen und/oder einen Wechsel wenigstens eines der beiden elektrischen Energiespeicher (4, 5) umfasst.

15. Blutpumpenanordnung umfassend:
eine Blutpumpe (2), und
eine Steuereinheit (1) gemäß einem der vorhergehenden Ansprüche; wobei die Blutpumpe mit der Steuereinheit über die Übertragungsschnittstelle (6a, 6b) elektrisch verbunden ist.
